# EUROPEAN PATENT SPECIFICATION

(11) **EP 2 214 644 B1**
(45) Date of publication and mention of the grant of the patent: **20.03.2013**
(21) Application number: 08848169.2
(22) Date of filing: 22.10.2008
(51) Int. Cl.: A61K 9/08, A61K 9/10, A61K 9/107, A61K 47/10, A61K 47/14, A61K 47/34, A61K 47/44, A61K 31/55, A61K 31/4709, A61K 9/00

(54) **WATER-IMMISCIBLE MATERIALS AS VEHICLES FOR DRUG DELIVERY**
WASSERUNLÖSLICHE MATERIALIEN ALS VEHIKEL ZUR WIRKSTOFFFREISETZUNG
MATIÈRES NON MISCIBLES À L'EAU EN TANT QUE VÉHICULES POUR L'ADMINISTRATION D'UN MÉDICAMENT

(30) Priority: 05.11.2007 US 985308 P
(43) Date of publication of application: 11.08.2010
(73) Proprietor: Bausch & Lomb Incorporated, Rochester, NY 14604-2701 (US)
(72) Inventor: ROHRS, Brian, R., Fairport NY 14450 (US); COFFEY, Martin, J., Pittsford NY 14534 (US)
(74) Representative: Glas, Holger
(86) International application number: PCT/US2008/080665
(87) International publication number: WO 2009/061607

(56) References cited:
- EP-A- 1 082 966
- WO-A-01/89495
- WO-A-94/05298
- WO-A-2008/021729
- WO-A-2008/027796
- WO-A-2008/154136
- WO-A-2009/042395
- US-A1- 2004 157 825
- US-A1- 2006 292 203
- PAWAR P K; MAJUMDAR D K: "In vitro permeation characteristics of moxifloxacin from oil drops through excised goat, sheep, buffalo and rabbit corneas" PHARMAZIE, vol. 62, no. 11, 2007, pages 853-857, XP002527016
- POPOVICI I ET AL: "Formulation et essais in vivo de collyres huileux d'indométacine" 19930101, vol. 3, no. 3, 1 January 1993 (1993-01-01), pages 266-270, XP002011711
- NELL BARBARA ET AL: "The effect of topical pimecrolimus on keratoconjunctivitis sicca and chronic superficial keratitis in dogs: results from an exploratory study" VETERINARY OPHTHALMOLOGY, vol. 8, no. 1, 2005, pages 39-46, XP002527017

## Description

### FIELD OF THE INVENTION

The present invention generally relates to water-immiscible materials as vehicles for drug delivery. More specifically, the present invention relates to methods of using such compositions for the treatment or control of ocular diseases or disorders.

### BACKGROUND OF THE INVENTION

In general, the outer elements of the eye comprise the lacrimal apparatus and the conjunctival sac. The eye also includes a number of other structures. For example, the sclera serves as the outer coating of the eyeball while a colored membrane called the iris regulates the entrance of light through the pupil, a contractile opening at the center of the iris that responds to light and darkness. The lens of the eye is a transparent refracting body that focuses light rays to form an image on the retina, which in turn receives and transmits them to the brain via the optic nerve. To nourish such structures and to assist with the removal of waste products, the aqueous humor, a fluid derived from the blood by a process of secretion and ultrafiltration through the ciliary processes circulates from the posterior chamber to the anterior chamber of the eye and leaves the eye through the trabecular network and Schlemm's canal. Lastly, eyelids and a mucous membrane that lines the eyelids known as the conjunctiva protect the eye and distribute tears. Thus, in light of such structural differentiation, the delivery of therapeutic ophthalmic components to the ocular environment can be very challenging.

Topical application is the most common route of administration of ophthalmic components. Advantages of such an application can include convenience, simplicity, noninvasive nature, and the ability of the patient to self-administer. For example, most topical ocular preparations are commercially available as solutions or suspensions that are applied directly to the eye via an applicator such as an eye dropper.

US Patent No. 5,480,914 and US Patent No. 5,620,699, both to Meadows, describe drop-instillable topical, nonaqueous thixotropic drug delivery vehicles containing a substantially homogeneous dispersion of at least one suspending aid in a nonaqueous perflourocarbon or fluorinated silicone liquid carrier for use in delivering ophthalmic components to aqueous physiological systems such as the eye. US Patent No. 3,767,788 to Rankin describes a drop-instillable ophthalmic solution containing an aqueous solution of polyethylene oxide, optionally polyethylene glycol, and other optional ingredients to lubricate and cushion eyes traumatized by contact lens wear.

Alternatively, ophthalmic components may be delivered topically to the eye via an ointment or gel. Such delivery vehicles prolong contact time with the external ocular surface and can offer extended dosing intervals such as "sustained release" type dosing. Ophthalmic components may also be delivered topically to the eye by devices such as contact lenses, cotton pledgets, or membrane-bound inserts.

Soft contact lenses can absorb water-soluble drugs and release them to the eye over prolonged periods of time whereas cotton pledgets (i.e., small pieces of cotton) can be saturated with ophthalmic solutions and placed in the conjunctival sac to topically deliver medicaments. A membrane-bound insert (e.g., Ocusert®) is a membrane-controlled drug delivery system. Following placement onto the bulbar conjunctiva under the upper or lower eyelid, the device releases ophthalmic medicaments slowly over time.

However, because of losses of the administered ophthalmic formulation through tear drainage, topically administered medicaments do not typically penetrate in useful concentrations to the posterior cavity of the eye, and therefore, are of little therapeutic benefit to treat or control diseases of the retina, optic nerve and other posterior segment structures. Additionally, some currently available topical delivery vehicles themselves have inherent disadvantages. For example, ointments may impede delivery of other ophthalmic components by serving as a barrier to contact. Ointments may also blur vision after administration. Moreover, the efficacy of ophthalmic medicaments in suspension, which are delivered via drop applicators, can be inconsistent due to easy settlement of the active ingredients from the suspension. As a result, proper administration technique frequently determines the efficacy of such medicaments.

Formulating techniques can also play a significant role in drug delivery and therapeutic outcomes in the ocular environment. Several ophthalmic components are poorly soluble in a variety of topical drug delivery vehicles, in turn, making delivery to the posterior cavity in an efficacious manner difficult. To overcome such difficulties associated with topical administration, ophthalmic components can be delivered to regions of the posterior cavity via ocular injection routes of administration. Thus, a number of ocular injection methodologies have been employed to deliver ophthalmic components.

US Patent No. 5,718,922 to Herrero-Vanrell et al., describes a method of forming microspheres containing a hydrophilic drug or agent for injection within the eye to provide localized treatment over a sustained period of time. Alternatively, US Patent No. 5,336,487 to Refojo et al., describes a method of treating an intraocular structural disorder of the retina by injecting a liquid silicone/fluorosilicone oil emulsion into the vitreous humor of the eye to treat the disorder and allow the retina to heal. However, such microspheres or emulsions may occlude the visual axis when delivered by an intravitreal injection.

Alternatively, US Patent No. 5,366,739 and US Patent No. 5,830,508, both to MacKeen, describe a composition and method for topical, prolonged delivery of a therapeutic agent to the eye for the treatment of dry eye syndrome. The therapeutic agent is further described as a water-soluble, calcium-based composition that is placed within a carrier, which is preferably hydrophobic/non-aqueous in nature (e.g., petrolatum or a combination of petrolatum and white wax). The composition is then delivered manually or by sterile cotton application to the extraocular skin adjacent to the lateral canthus of the eye. Although non-aqueous delivery vehicles are described for topical application for extraocular usage, injectable compositions and methods are not disclosed.

The article "Formulation et essais in vivo de collyres huileux d'indométacine" by Popovici et al (vol.3, no.3, 1 January 1993, p. 266-270; S.T.P. Pharma Sciences) relates to indomethracin containing eye drops. Further, a review of solubilizing excipients for oral and injectable formulations by Robert G. Strickley describes such agents as including water-soluble solvents (e.g., polyethylene glycol 300), non-ionic surfactants (polysorbate 80), water-soluble lipids (e.g., castor oil), organic liquids/semi-solids (e.g., beeswax), and various cyclodextrins and phospholipids. See R.G. Strickley, Solubilizing Excipients in Oral and Injectable Formulations, Pharmaceutical Research, Vol. 21, No. 2, pp. 201-30 (Feb. 2004). However, ocular injectable formulations, especially extended, controlled or sustained release-based formulations for injection into the posterior regions of the ocular environment are not disclosed.

As discussed above, delivering therapeutic compounds to the ocular environment can be challenging. Therefore, while medicaments are currently available to treat ocular diseases, there still is a need for improved ophthalmic compositions and methods for delivering such compositions to the posterior regions of the ocular environment, especially to achieve an extended, controlled or sustained release of the active ingredients of such compositions. Novel and improved compositions can significantly overcome existing difficulties in providing therapeutically effective amounts of the pharmaceutical components to the targeted tissues.

### SUMMARY OF THE INVENTION

The present invention provides pharmaceutical compositions as defined in claim 1 for use in the treatment of an ocular disease or disorder by injection into the vitreous cavity or the subconjunctiva of an eye.

The present invention provides an injectable ophthalmic composition, having at least one pharmaceutical component and at least one water-immiscible material, such that the pharmaceutical component and the water-immiscible material can be combined to form at least one mixture suitable for formulation for ocular injection.

The pharmaceutical component is selected from anti-inflammatory agents, anti-infective agents (including antibacterial, antifungal, antiviral, antiprotozoal agents), anti-angiogenic agents, and alpha-adrenergic receptor blockers.

The inventive mixture comprising a pharmaceutical component and a water-immiscible vehicle is suitable for ocular injection.

In still another aspect, the mixture has a viscosity in the range from about 10 centipoises ("cp" or mPa.s) to about 10,000 cp.

In yet another aspect, the pharmaceutical component present in the mixture at a concentration between about 0.01% (by weight) to about 50% (by weight) and the water-immiscible vehicle is present in the mixture at a concentration between about 99.99% (by weight) to about 50% (by weight) of the total weight of the mixture. Throughout this disclosure, unless otherwise specified, concentrations of an ingredient of the composition or formulation are in weight percent. In certain embodiments, the concentration of a pharmaceutical component is in the range from about 0.1% to about 25% (or alternatively, from about 0.1 % to about 10%, or from about 0.1% to 5%, or from about 0.1 % to about 2%, or from about 0.1 % to 1%, or from about 0.5% to about 5%, or from about 0.5% to about 2%, or from about 0.2% to about 2%, or from about 0.2% to 1%). In certain other embodiments, the water-immiscible vehicle constitutes substantially the balance of the mixture (other than the presence of possible minor amounts of other additives that may be included in the mixtures).

In another aspect of the present invention, the mixture can be a suspension containing particles of the pharmaceutical component in the water-immiscible material. In an embodiment for this particular aspect of the present invention, the particles of the pharmaceutical component have a particle size of between about 0.01 µm to about 1 µm in diameter. In another embodiment, the particle size is between about 0.05 µm to about 0.5 µm in diameter.

In another aspect of the present invention, the mixture can be formulation for a sustained-release, controlled release, or extended release of the pharmaceutical component and releases the pharmaceutical component over a period of 1 year, 6 months, 90 days or greater, 30 days or greater, 24 hours or greater, 12 hours or greater, or 8 hours or greater.

The inventive mixture is suitable for formulation for ocular injection and is capable of being injected into, for example, the vitreous humor or the subconjunctiva areas of a human or animal eye.

Moreover, it shall also be appreciated that a formulation or composition of the present invention having the controlled release, sustained release, or extended release characteristics noted herein can be incorporated into an ophthalmic device or implant.

According to the present invention there is provided a method of treating or controlling an ocular disease or disorder. The method comprises administering into the vitreous cavity or subconjunctiva of an eye a mixture comprising at least one pharmaceutical component and at least one water-immiscible material. The ocular disease or disorder can include, but are not limited to, a posterior-segment disease or disorder. In certain embodiments, such disease or disorder is selected from the group consisting of diabetic retinopathy, diabetic macular edema, cystoid macular edema, age macular degeneration (including the wet and dry form), optic neuritis, retinitis, chorioretinitis, intermediate and posterior uveitis, choroidal neovascuralization, and combinations thereof.

In a further aspect, any composition or formulation and method of the present invention can provide enhanced concentrations of pharmaceutical active ingredients at an ocular target tissue. The target tissue is the vitreous cavity or subconjunctiva that is difficult to treat with topically applied medicaments.

These and other features and advantages of the present invention will be further understood and appreciated by those skilled in the art by reference to the following detailed description and claims.

### DETAILED DESCRIPTION OF THE INVENTION

As used herein, the term "control" also includes reduction, amelioration, alleviation, and prevention.

The present invention provides an injectable ophthalmic composition as defined in claim 1, having at least one pharmaceutical component and at least one water-immiscible material, such that the pharmaceutical component and the water-immiscible material can be combined to form at least one mixture suitable for formulation for ocular injection.

The pharmaceutical component is solubilizable in the water-immiscible material in an amount of at least about 0.1 mg/g. In another embodiment, the pharmaceutical component is solubilizable in the water-immiscible material in an amount in the range from about 0.1 mg/g to about 200 mg/g. Alternatively, the pharmaceutical component is solubilizable in the water-immiscible material in an amount in the range from about 0.1 mg/g to about 100 mg/g, or from about 0.1 mg/g to about 75 mg/g, or from about 0.1 mg/g to about 50 mg/g, or from about 0.1 mg/g to about 25 mg/g, or from about 0.1 mg/g to about 10 mg/g, or from about 1 mg/g to about 200 mg/g, or from about 1 mg/g to about 100 mg/g, or from about 1 mg/g to about 50 mg/g, or from about 1 mg/g to about 25 mg/g, or from about 1 mg/g to about 10 mg/g. Such solubility is measured at a physiological pH (about 7.4) and at about 25° C.

The present invention provides a composition comprising, and a method for delivering, a pharmaceutical component that is insoluble or exhibits low solubility in water. As used herein, the phrase "low solubility in water" or "poorly water soluble" means solubility in water of less than 0.1 mg/g at physiological pH (about 7.4) and about 25° C. Using a water-immiscible material that forms a separate phase from the substantially aqueous ocular environment can improve the potential for localization of particles of one or more of the pharmaceutical components that can be used in various aspects of the present invention. Without being bound by a particular theory, it is believed that if particles of the pharmaceutical component are suspended within the separate phase, they are less likely to migrate into the visual axis and impair vision. Thus, the disadvantages associated with prior-art topical suspensions and ointments are lessened or prevented.

A variety of pharmaceutical components, especially ophthalmic pharmaceutical components, known within the pharmaceutical industry are suitable for use in various aspects of the present invention. Pharmaceutical components are those mentioned in claim 1 and utilized in treating ocular indications, diseases, disorders, conditions, syndromes, injuries, and the like. Additionally, although not wanting to be bound by any particular theory, it is believed that the present invention is particularly suited for use with pharmaceutical components that are water insoluble or poorly water soluble, but are solubilizable in water-immiscible materials. Thus, the present invention enhances the delivery to and bioavailability at a target tissue of such insoluble or poorly soluble pharmaceutical components.

Inventive pharmaceutical components include anti-inflammatory agents, anti-infective agents (including antibacterial, antifungal, antiviral, antiprotozoal agents), anti-angiogenic agents, alpha-adrenergic receptor blockers.

In another embodiment, the pharmaceutical component is selected from the group consisting of anti-inflammatory agents.

In one embodiment, the pharmaceutical component comprises a fluoroquinolone having Formula I (a new-generation fluoroquinolone antibacterial agent, disclosed in US Patent No. 5,447,926 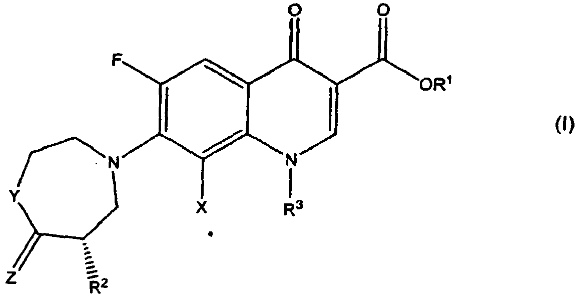 wherein R¹ is selected from the group consisting of hydrogen, unsubstituted lower alkyl groups, substituted lower alkyl groups, cycloalkyl groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, and groups that can be hydrolyzed in living bodies; R² is selected from the group consisting of hydrogen, unsubstituted amino group, and amino groups substituted with one or two lower alkyl groups; R³ is selected from the group consisting of hydrogen, unsubstituted lower alkyl groups, substituted lower alkyl groups, cycloalkyl groups, unsubstituted lower alkoxy groups, substituted lower alkoxy groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, unsubstituted C₅-C₂₄ aryloxy groups, substituted C₅-C₂₄ aryloxy groups, unsubstituted C₅-C₂₄ heteroaryloxy groups, substituted C₅-C₂₄ heteroaryloxy groups, and groups that can be hydrolyzed in living bodies; X is selected from the group consisting of halogen atoms; Y is selected from the group consisting of CH₂, O, S, SO, SO₂, and NR⁴, wherein R⁴ is selected from the group consisting of hydrogen, unsubstituted lower alkyl groups, substituted lower alkyl groups, and cycloalkyl groups; and Z is selected from the group consisting of oxygen and two hydrogen atoms.

In another embodiment, the pharmaceutical component comprises a fluoroquinolone having Formula II. 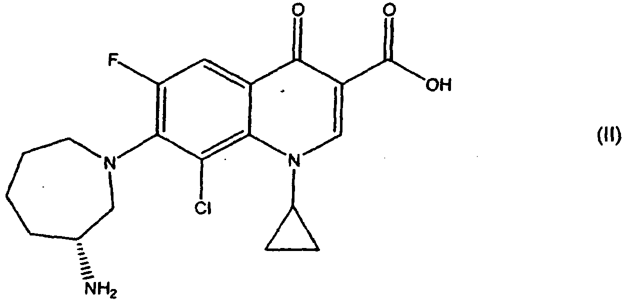 ((R)-(+)-7-(3-amino-2,3,4,5,6,7-hexahydro-1H-azepin-1-yl)-8-chloro-1-cyclopropyl-6-fluoro-1,4-dihydro-4-oxoquinoline-3-carboxylic acid).

In still another embodiment, the pharmaceutical component comprises a glucocorticoid receptor agonist having Formulae III or IV, as disclosed in US Patent Application Publication 2006/0116396 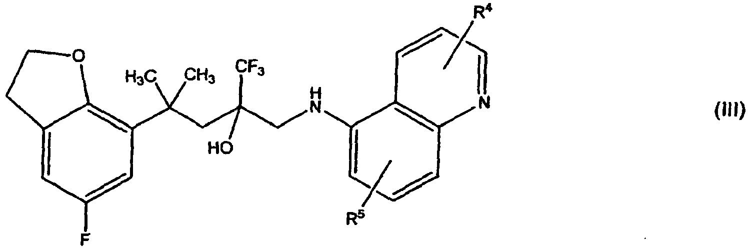 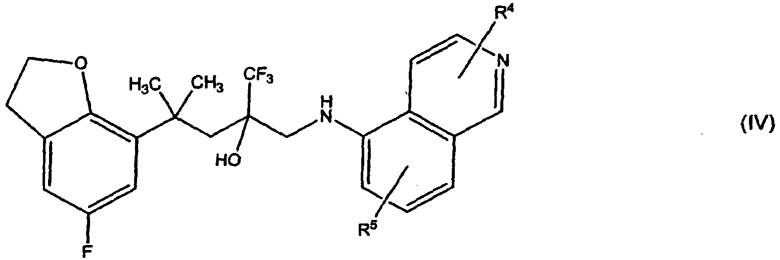 wherein R⁴ and R⁵ are independently selected from the group consisting of hydrogen, halogen, cyano, hydroxy, C₁-C₁₀ (alternatively, C₁-C₅ or C₁-C₃) alkoxy groups, unsubstituted C₁-C₁₀ (alternatively, C₁-C₅ or C₁-C₃) linear or branched alkyl groups, substituted C₁-C₁₀ (alternatively, C₁-C₅ or C₁-C₃) linear or branched alkyl groups, unsubstituted C₃-C₁₀ (alternatively, C₃-C₆ or C₃-C₅) cyclic alkyl groups, and substituted C₃-C₁₀ (alternatively, C₃-C₆ or C₃-C₅) cyclic alkyl groups.

In yet another embodiment, the pharmaceutical component comprises a glucocorticoid receptor agonist having Formula V (a species of compound having Formula III).

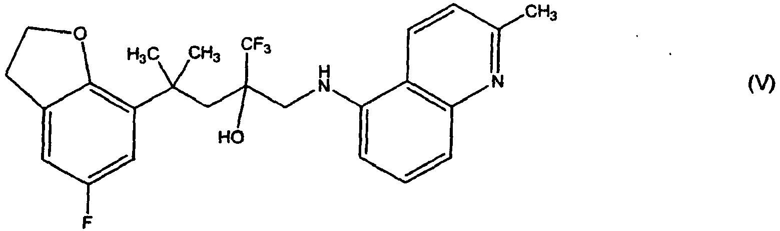

Suitable water-immiscible materials for use in the present invention include, but are not limited to, castor oil, corn oil, mineral oil, miglyol, benzyl benzoate, polycaprolactone, poly(caprolactone) triol, ethyl oleate, vitamin A, α-tocopherol (vitamin E), medium-chain triglyceride, long-chain triglyceride, derivatives thereof, or combinations thereof.

A viscosity-modifying compound can be designed to facilitate the administration of the composition into the subject or to promote the bioavailability in the subject for the intended time period of treatment. A viscosity-modifying compound can be a low or high molecular weight material, depending on the viscosity of the water-immiscible carrier used. A non-limiting example of a low molecular weight viscosity-modifying agent is a medium-chain triglyceride ("MCT"), wherein the fatty acyl moiety comprises 4-12 carbon atoms. A viscosity-modifying compound can be a pharmaceutically acceptable polymer of suitable molecular weight and may be chosen so that the composition is not readily dispersed after being administered into the vitreous. Such compounds may enhance the viscosity of the composition, and include, but are not limited to: long-chain triglycerides ("LCT," wherein the fatty acyl moiety has more than 12, preferably more than 18, and more preferably more than 22, carbon atoms), water-immiscible acrylic ester polymers, polysiloxanes, and water-immiscible polypeptides.

In one aspect, the viscosity of the composition or formulation is in the range from about 10 cp to about 10,000 cp. Alternative the viscosity of the composition or formulation is in the range from about 10 cp to about 5,000 cp, or from about 10 cp to about 2,000 cp, from about 10 cp to about 1,000 cp.

The mixture can be a sustained-release, controlled release or extended release composition that releases the pharmaceutical component over a period of time. In a preferred embodiment, the mixture can release the pharmaceutical component over a period of 8 hours or greater. In another preferred embodiment, the mixture can release the pharmaceutical component over a period of 12 hours or greater. In further preferred embodiment, the mixture can release the pharmaceutical component over a period of 24 hours or greater. In a still further preferred embodiment, the mixture can release the pharmaceutical component over a period of 30 days or greater. In yet a still further preferred embodiment, the mixture can release the pharmaceutical component over a period of 90 days or greater, or at least 6 months, or at least one year.

The mixture is formulated for injection into an ocular environment, namely the vitreous cavity or the subconjunctiva of an eye within a human or an animal. The mixture can be formulated for ocular injection according to known methods and principles, and then injected using an injection delivery device such as an appropriately gauged needle; for example, 25-30 gauge needle.

Optionally, before the mixture is injected into an ocular environment, the mixture can be sterilized. Suitable methods of sterilization include, but are not limited to, sterile filtration, thermal sterilization, and gamma irradiation. Where sterile filtration is selected, one suitable method of sterile filtration can utilize a filter having a pore size of at least about 0.2 microns or less. Where thermal sterilization is selected, one suitable method of thermal sterilization can include sterilizing the mixture at a temperature of at least about 150 °C for a period of at least about 25 minutes. Where gamma irradiation is selected, one suitable method can include exposure of the compositions of the present invention to gamma rays at a level of from about 2.5 Mrad to about 3.5 Mrad.

The present invention refers to treating an ocular disease, disorder, or condition. The treatment includes administering at least one mixture comprising at least one pharmaceutical component and at least one water-immiscible material into an ocular environment. The mixture can be used to treat an ocular disease, disorder, or including, but not limited to, diabetic retinopathy, diabetic macular edema, cystoid macular edema, age macular degeneration (including the wet and dry form), optic neuritis, retinitis, chorioretinitis, intermediate and posterior uveitis, choroidal neovascuralization, and combinations thereof.

The water-immiscible material and the pharmaceutical component and the water-immiscible vehicle can be combined to form any suitable mixture, including, but not limited to, a hydrophobic solution, a semi-solid, or a suspension. For example, the mixture can be a suspension containing particles of the pharmaceutical component in the water-immiscible material. In various embodiments of the present invention, the particles of the pharmaceutical component have a particle size of between about 0.01 µm to about 1 µm in diameter. In another embodiment, the particle size is between about 0.05 µm to about 0.5 µm in diameter.

Although not wanting to be bound by any particular theory, Applicant believes that the water-immiscible material as a drug delivery vehicle of the present invention can address one or more of the challenges described herein regarding the delivery of pharmaceutical components to target tissues within the ocular environment.

For example, solubilization of a pharmaceutical component that has a low solubility in an aqueous or water-miscible material may have a higher solubility in a water-immiscible material. Such increased solubility can enhance the delivery of that pharmaceutical component or component particles to those target tissues, and thereby enhance the component's concentration at, in, or near the target tissue.

In some instances, the amount or dose of the pharmaceutical component can be completely soluble in the water-immiscible compound such that the entire amount or dose is delivered as a water-immiscible solution to the desired ocular environment. In other instances, the component can be delivered as a suspension, yet because of the higher solubility in the water-immiscible delivery vehicle of the present invention, particle dissolution rate is faster than that achievable in an aqueous vehicle. Therefore, delivery of a pharmaceutical component is less dependent on particle size and the desired concentration of the pharmaceutical component in target tissues is achieved more readily.

For sustained release, controlled release, or extended release delivery applications, the water-immiscible formulation or composition of the present invention, upon administration (e.g., by injection), can form a droplet. Such a droplet provides a localization of the active pharmaceutical ingredient at the desired site of treatment, and thus has advantage over a formulation or composition that is readily dispersed shortly after administration. Additionally, the affinity of the solubilized pharmaceutical component may be greater, and therefore, the amount of the component available to the surrounding environment can be greater.

In other instances, the water-immiscible material may inhibit suspended particles from settling out of the water-immiscible phase and into the aqueous ocular environment. In further instances, the water-immiscible material may provide lower solubilization for a hydrophilic pharmaceutical component or additional additive of the composition such that dissolution of suspended particles may be slower than if exposed to aqueous media. In all of these instances, the effect can be a sustained release, controlled release, or extended release of a pharmaceutical component from the water-immiscible material.

An additional advantage of using a water-immiscible material that forms a separate phase from the aqueous ocular environment is the improved potential for localization of particles of one or more of the pharmaceutical components that can be used in accordance with the present invention. Without being bound by any particular theory, Applicant believes that if particles of the pharmaceutical component are suspended within the separate phase, they are less likely to migrate into the visual axis and occlude vision. Thus, the disadvantages associated with topical suspensions and ointments are lessened or prevented

### Examples

The following illustrative examples provide further description and insight regarding one or more aspects and/or embodiments of the present invention.

### Comparative EXAMPLE 1

Water-Immiscible Solution Formulation Containing Anti-inflammatory Drug
70% castor oil
25% medium chain triglycerides
2% sorbitan laurate (Span 20, available from, for example, Sigma Aldrich)
2% sorbitan oleate (Span 80, available from, for example, Sigma Aldrich)
1 % α-tocopherol

Add all the components to the castor oil and mix to give a uniform mixture. Then add a 5-substituted quinoline anti-inflammatory drug disclosed in US Patent Application Publication 2006/0116396 to saturate the mixture and form the final formulation. The formulation is suitable for being administered to the posterior cavity to provide sustained release of the anti-inflammatory drug for the treatment of a posterior inflammatory condition.

### Comparative EXAMPLE 2

Water-Immiscible Suspension Formulation Comprising Brimonidine
70% castor oil
25% medium chain triglycerides
2% sorbitan laurate (Span 20, available from Sigma Aldrich)
2% sorbitan oleate (Span 80, available from Sigma Aldrich)
1% α-tocopherol
brimonidine free base

Add all the components except brimonidine free base to the castor oil to produce an oil mixture. Add the desired amount of brimonidine free base to a small portion of oil mixture such that the brimonidine free base concentration is 100-500 mg/mL. Use wet milling to reduce the average particle size of the pharmaceutical component to 10 µm or less. Dilute this milled suspension to the desired brimonidine free base concentration with additional oil mixture to produce the final formulation, which can be administered into the vitreous cavity for the long-term control of risk of optic nerve degeneration in patients having symptoms of glaucoma.

### Comparative EXAMPLE 3

Pemulen®-Based Emulsion Containing Moxifloxacin
10% castor oil
0.15% acrylates/C10-30 alkyl acrylate cross-linked polymer (Pemulen® TR-1, available from Lubrizol Corp.)
0.15% sorbitan laurate (Span 20, available from Sigma Aldrich)
5% propylene glycol
0.1% EDTA disodium
0.1% sodium ascorbate
0.1% α-tocopherol
0.5% phenylethyl alcohol
q.s. moxifloxacin (a fluoroquinolone antibacterial drug) to saturate castor oil phase
q.s. NaOH to adjust pH to 5.5-6.0
q.s. water

Dissolve moxifloxacin in castor oil to saturate the oil phase. Add sorbitan laurate and tocopherol to the oil phase and mix. Disperse the acrylates/C10-30 alkyl acrylate cross-linked polymer in the oil phase. Add the remaining ingredients to the water and mix until uniform. Add the water phase to the oil phase and mix with high shear for about 15-30 minutes to form an emulsion. Add NaOH to adjust pH to about 5.5-6.0. The emulsion may be administered into a patient having bacteria infection of the posterior cavity.

### Comparative EXAMPLE 4

Emulsion Containing Anti-angiogenic Agent
10% castor oil
4% polysorbate 80 (Tween 80, available from Acros Organics in Geel, Belgium)
5% propylene glycol
0.1% EDTA disodium
0.1% sodium ascorbate
0.1% α-tocopherol
0.5% phenylethyl alcohol
q.s. Lucentis^{®} (ranibizumab, an ophthalmic anti-angiogenic agent) to saturate castor oil phase
q.s. NaOH or HCl to adjust pH to 5.5-6.0
q.s water

Dissolve Lucentis^{®} in the castor oil to saturate the oil phase. Add polysorbate 80 and tocopherol to the oil phase and mix. Add the remaining ingredients to the water and mix until uniform. Add the water phase to the oil phase and mix with high shear for about 15-30 minutes to form an emulsion. Add NaOH or HCl to adjust pH to about 5.5-6.0. The emulsion may be administered into the posterior cavity for the long-term treatment of posterior angiogenesis condition such as diabetic retinopathy.

### EXAMPLE 5

Water-Immiscible Solution Formulation Containing COX-2 Inhibitor
castor oil
q.s. celecoxib (known by the tradename Celebrex^{®}, a COX-2 inhibitor) to saturate the formulation

Add the pharmaceutical component to the castor oil. Mix until uniform to produce a water-immiscible solution that may be used a long-term treatment of a posterior condition having etiology in inflammation.

### EXAMPLE 6

Water-Immiscible Suspension Formulation Containing Antibacterial Drug
castor oil
q.v. (R)-(+)-7-(3-amino-2,3,4,5,6,7-hexahydro-1H-azepin-1-yl)-8-chloro-1-cyclopropyl-6-fluoro-1,4-dihydro-4-oxoquinoline-3-carboxylic acid (a fluoroquinolone antibacterial agent, disclosed in US Patent No. 5,447,926)

Add desired amount of the fluoroquinolone antibacterial agent to a small portion of the castor oil such that the pharmaceutical component concentration is 100-500 mg/mL. Use wet milling to reduce the average particle size of the pharmaceutical component to 10 µm or less. Dilute this milled suspension to the desired pharmaceutical component concentration with additional castor oil to produce a suspension, which may be administered into the vitreous cavity to treat posterior chamber infection.

### EXAMPLE 7

Water-Insoluble Suspension Formulation
98% poly(caprolactone) triol (molecular weight approximately 900)
2% loteprednol etabonate

Dry mill loteprednol etabonate to reduce particle size. Weigh the total loteprednol etabonate amount and transfer into the mixing vessel. Add the poly(caprolactone) triol (molecular weight approximately 900) in an amount such that loteprednol etabonate amount is 2 wt/vol % and continue mixing for at least 15 minutes to produce the final suspension formulation, which may be used to treat ocular inflammation. This suspension formulation is equally suitable for topical administration and for intravitreal injection.

### EXAMPLE 8

Water-Insoluble Suspension Formulation
74% poly(caprolactone) triol (molecular weight approximately 900)
25% poly(caprolactone) triol (molecular weight approximately 300)
1% brimonidine free base

Mix the poly(caprolactone) triol (molecular weight approximately 900) and poly(caprolactone) triol (molecular weight approximately 300) in a ratio of 74.75:25.25 V:V in an amount such that the amount of brimonidine free base is 2 wt/vol %. Dry mill the brimonidine free base to reduce particle size. Weigh the total brimonidine free base amount and transfer into the new mixing vessel. Add the poly(caprolactone) blend in an amount so the amount of brimonidine free base is 1 wt/vol % to produce the final suspension formulation, which may be administered intravitreally to stop the progression of glaucomatous neurodegeneration.

### EXAMPLE 9

Water-Insoluble Polycaprolatone Formulation Containing Anti-angiogenic Agent

A solution formulation comprising polycaprolactone triol 300 and a water-insoluble anti-angiogenic agent at a concentration of 2 mg/mL was prepared and administered intravitreally in 12 New Zealand pigmented rabbits. Each rabbit received 50 µl of the formulation at a target dosage of 100 µg. After 56 days, 43% of the amount of anti-angiogenic agent originally administered remained in the vitreous, demonstrating the sustained release property of the formulation.

## Claims

1. An injectable ophthalmic pharmaceutical composition for use in the treatment of an ocular disease or disorder by injection into the vitreous cavity or the subconjunctiva of an eye, the composition consisting of:
at least one pharmaceutical component;
at least one water-immiscible material; and
optionally a viscosity-modifying compound, wherein the viscosity-modifying compound is selected from medium-chain triglycerides, long-chain triglycerides, water-immiscible acrylic ester polymers, polysiloxanes, or water-immiscible polypeptides:
wherein the pharmaceutical component is selected from anti-inflammatory agents, anti-infective agents, alpha-adrenergic receptor blockers and anti-angiogenic agents:
wherein the pharmaceutical component has a solubility in water of less than 0.1 mg/g measured at a pH of 7.4 and at 25° C, the pharmaceutical component is solubilizable in the water-immiscible material in an amount of at least 0.1 mg/g measured at a pH of 7.4 and at 25° C;
and wherein the pharmaceutical component and the water-immiscible material are combined to form at least one mixture suitable for formulation for ocular injection.

2. The composition for use according to claim 1, wherein the pharmaceutical component is solubilizable in the water-immiscible material in an amount in the range from 0.1 mg/g to 200 mg/g at a pH of 7.4 and at 25° C.

3. The composition for use according to claim 1, wherein the water-immiscible material is selected from the group consisting of castor oil, corn oil, mineral oil, miglyol, benzyl benzoate, polycaprolactone, poly(caprolactone) triol, ethyl oleate, vitamin A, α-tocopherol (vitamin E), medium-chain triglyceride, long-chain triglyceride, and combinations thereof.

4. The composition for use according to claim 1, wherein the pharmaceutical component represents between 0.01% and 50%, and the water-immiscible material represents between 99.99% and 50%, based upon the total weight of the composition.

5. The composition for use according to claim 1, wherein the pharmaceutical component represents between 0.1% and 25% of the total weight of the composition

6. The composition for use according to claim 1, wherein the composition is a suspension containing particles of the pharmaceutical component in the water-immiscible material.

7. The composition for use according to claim 6, wherein the particles of the pharmaceutical component have a particle size of between 0.01 µm to 1 µm in diameter.

8. The composition for use according to claim 1, wherein the composition provides a sustained-release, controlled release, or extended release of the pharmaceutical component over a period of 90 days or greater at a target tissue.

9. The injectable ophthalmic composition for use according to claim 1, for providing extended availability of a pharmaceutical component in an ocular environment of a subject for an extended treatment of an ocular disorder in said subject.

10. The composition for use according to claim 9, wherein the mixture is injectable into a vitreous humor of the ocular environment.

11. The composition for use according to claim 9, wherein the mixture is injectable into a subconjunctiva of the ocular environment.

12. The composition for use according to claim 9, wherein said ocular disorder is selected from the group consisting of diabetic retinopathy, diabetic macular edema, cystoid macular edema, wet and dry age macular degeneration, optic neuritis, retinitis, chorioretinitis, intermediate and posterior uveitis, choroidal neovascuralization, and combinations thereof.

## Patentansprüche

1. Injizierbare pharmazeutische ophthalmische Zusammensetzung zur Verwendung in der Behandlung von Augenerkrankungen oder Augenfunktionsstörungen mittels Injektion in den Glaskörper oder die Netzhaut des Auges, wobei die Zusammensetzung besteht aus:
mindestens einer pharmazeutischen Komponente;
mindestens einem nicht mit Wasser mischbaren Stoff; und
wahlweise einer viskositätsverändernden Verbindung, wobei die viskositätsverändernde Verbindung ausgewählt ist aus Triglyceriden mittlerer Kettenlänge, langkettigen Triglyceriden, nicht mit Wasser mischbaren Acrylesterpolymeren, Polysiloxanen, oder nicht mit Wasser mischbaren Polypeptiden;
wobei die pharmazeutische Komponente ausgewählt ist aus Entzündungshemmern, Antünfektiva, alpha-adrenergen Rezeptorblockern und Angiogenesehemmern;
wobei die pharmazeutische Komponente eine Löslichkeit in Wasser von weniger als 0.1 mg/g bei pH 7.4 und 25°C aufweist, eine Menge von mindestens 0.1 mg/g bei pH 7.4 und 25°C der pharmazeutischen Komponente in dem nicht mit Wasser mischbaren Stoff löslich sind; und
wobei die pharmazeutische Komponente und der nicht mit Wasser mischbare Stoff gemischt werden, um mindestens eine Mischung zu bilden, die als Formulierung zur Injektion in das Auge geeignet ist.

2. Zusammensetzung zur Verwendung gemäß Anspruch 1, wobei die pharmazeutische Komponente in Mengen im Bereich zwischen 0.1 mg/g und 200 mg/g bei pH 7.4 und 25°C in dem nicht mit Wasser mischbaren Stoff löslich ist.

3. Zusammensetzung zur Verwendung gemäß Anspruch 1, wobei der nicht mit Wasser mischbare Stoff ausgewählt ist aus der Gruppe bestehend aus Ricinusöl, Maiskeimöl, Mineralöl, Miglyol, Benzoesäurebenzylester, Polycaprolacton, Poly(caprolacton)triol, Ölsäureethylester, Vitamin A, α-Tocopherol (Vitamin E), Triglyceriden mittlerer Kettenlänge, langkettigen Triglyceriden, und Kombinationen hiervon.

4. Zusammensetzung zur Verwendung gemäß Anspruch 1, wobei zwischen 0.01% und 50% pharmazeutische Komponente, und zwischen 99.99% und 50% nicht mit Wasser mischbarer Stoff, bezogen auf das Gesamtgewicht der Zusammensetzung, enthalten sind.

5. Zusammensetzung zur Verwendung gemäß Anspruch 1, wobei zwischen 0.1% und 25% pharmazeutische Komponente, bezogen auf das Gesamtgewicht der Zusammensetzung, enthalten sind.

6. Zusammensetzung zur Verwendung gemäß Anspruch 1, wobei die Zusammensetzung eine Suspension ist, die Partikel der pharmazeutischen Komponente in dem nicht mit Wasser mischbaren Stoff enthält.

7. Zusammensetzung zur Verwendung gemäß Anspruch 6, wobei die Partikel der pharmazeutischen Komponente eine Partikelgröße zwischen 0.01 µm und 1 µm Durchmesser aufweisen.

8. Zusammensetzung zur Verwendung gemäß Anspruch 1, wobei die Zusammensetzung eine verzögerte Freisetzung, eine kontrollierte Freisetzung oder eine verlängerte Freisetzung der pharmazeutischen Komponente in einem Zielgewebe über einen Zeitraum von 90 Tagen oder mehr ermöglicht.

9. Injizierbare ophthalmische Zusammensetzung zur Verwendung gemäß Anspruch 1, die eine verlängerte Verfügbarkeit einer pharmazeutischen Komponente in der Umgebung des Auges einer Person bei einer verlängerten Behandlung einer Augenerkrankung oder einer Augenfunktionsstörung der Person ermöglicht.

10. Injizierbare ophthalmische Zusammensetzung zur Verwendung gemäß Anspruch 9, wobei die Mischung in den Glaskörper des Auges injizierbar ist.

11. Injizierbare ophthalmische Zusammensetzung zur Verwendung gemäß Anspruch 9, wobei die Mischung in die Bindehaut des Auges injizierbar ist.

12. Injizierbare ophthalmische Zusammensetzung zur Verwendung gemäß Anspruch 9, wobei die genannte Augenerkrankung ausgewählt ist aus der Gruppe bestehend aus diabetischer Retinopathie, diabetischem Makulaödem, zystischem Makulaödem, feuchter und trockener altersbedingter Makuladegeneration, Sehnerventzündung, Netzhautentzündung, Chorioretinitis, Uveitis intermediate und posterior, choroidalen Gefäßneubildungen und Kombinationen hiervon.

## Revendications

1. Composition pharmaceutique injectable à usage ophtalmique pour utilisation dans le traitement d'une maladie ou d'un trouble oculaire par injection dans la cavité du corps vitré ou sous la conjonctive d'un oeil, la composition étant constituée de :
au moins un composant pharmaceutique ;
au moins un matériau non miscible avec l'eau ; et
éventuellement un composé modificateur de viscosité,
dans laquelle le composé modificateur de viscosité est choisi parmi les triglycérides à chaîne moyenne, les triglycérides à longue chaîne, les polymères d'esters acrylates non miscibles avec l'eau, les polysiloxanes, ou les polypeptides non miscibles avec l'eau ;
dans laquelle le composant pharmaceutique est choisi parmi les agents anti-inflammatoires, les agents anti-infectieux, les alpha-bloquants et les agents anti-angiogenèse ;
dans laquelle le composant pharmaceutique a une solubilité dans l'eau inférieure à 0,1 mg/g, mesurée à un pH de 7,4 et à 25°C, le composant pharmaceutique pouvant être solubilisé dans le matériau non miscible avec l'eau en une quantité d'au moins 0,1 mg/g, mesurée à un pH de 7,4 et à 25°C ;
et dans laquelle le composant pharmaceutique et le matériau non miscible avec l'eau sont combinés pour former au moins un mélange adapté à une formulation pour injection oculaire.

2. Composition pour utilisation selon la revendication 1, dans laquelle le composant pharmaceutique peut être solubilisé dans le matériau non miscible avec l'eau en une quantité située dans la plage allant de 0,1 mg/g à 200 mg/g à un pH de 7,4 et à 25°C.

3. Composition pour utilisation selon la revendication 1, dans laquelle le matériau non miscible avec l'eau est choisi dans le groupe constitué par l'huile de ricin, l'huile de maïs, l'huile minérale, le miglyol, le benzoate de benzyle, la polycaprolactone, le poly(caprolactone)triol, l'oléate d'éthyle, la vitamine A, l'□-tocophérol (vitamine E), les triglycérides à chaîne moyenne, les triglycérides à longue chaîne, et leurs combinaisons.

4. Composition pour utilisation selon la revendication 1, dans laquelle le composant pharmaceutique représente entre 0,01 % et 50 %, et le matériau non miscible avec l'eau représente entre 99,99 % et 50 % par rapport au poids total de la composition.

5. Composition pour utilisation selon la revendication 1, dans laquelle le composant pharmaceutique représente entre 0,1 % et 25 % du poids total de la composition.

6. Composition pour utilisation selon la revendication 1, laquelle composition est une suspension contenant des particules du composant pharmaceutique dans le matériau non miscible avec l'eau.

7. Composition pour utilisation selon la revendication 6, dans laquelle les particules du composant pharmaceutique ont une granulométrie correspondant à un diamètre compris entre 0,01 µm et 1 µm.

8. Composition pour utilisation selon la revendication 1, laquelle composition permet une libération soutenue, une libération contrôlée, ou une libération prolongée du composant pharmaceutique sur une période de 90 jours ou plus au niveau du tissu cible.

9. Composition injectable à usage ophtalmique pour utilisation selon la revendication 1, destinée à permettre une disponibilité prolongée d'un composant pharmaceutique dans un environnement oculaire chez un sujet pour un traitement prolongé d'un trouble oculaire chez ledit sujet.

10. Composition pour utilisation selon la revendication 9, dans laquelle le mélange est injectable dans l'humeur vitrée de l'environnement oculaire.

11. Composition pour utilisation selon la revendication 9, dans laquelle le mélange est injectable sous la conjonctive de l'environnement oculaire.

12. Composition pour utilisation selon la revendication 9, dans laquelle ledit trouble oculaire est choisi dans le groupe constitué par la rétinopathie diabétique, l'oedème maculaire diabétique, le syndrome d'Irvine, la dégénérescence maculaire exsudative ou atrophique liée à l'âge, la névrite optique, la rétinite, la choriorétinite, l'uvéite intermédiaire ou postérieure, la néovascularisation choroïdienne, et leurs combinaisons.
